# EUROPEAN PATENT SPECIFICATION

(11) **EP 3 393 278 B1**
(45) Date of publication and mention of the grant of the patent: **16.02.2022**
(21) Application number: 16795085.6
(22) Date of filing: 15.11.2016
(51) Int. Cl.: A24F 15/015, A24F 40/10, A24F 40/485, A24F 40/48

(54) **AN ELECTRICALLY OPERATED AEROSOL-GENERATING SYSTEM WITH A LIQUID PUMP**
ELEKTRISCH BETRIEBENES AEROSOL-ERZEUGUNGSSYSTEM MIT EINER FLÜSSIGKEITSPUMPE
SYSTÈME DE GÉNÉRATION D'AÉROSOL ACTIONNÉ ÉLECTRIQUEMENT AVEC UNE POMPE DE LIQUIDE

(30) Priority: 22.12.2015 EP 15202075
(43) Date of publication of application: 31.10.2018
(73) Proprietor: Philip Morris Products S.A., 2000 Neuchâtel (CH)
(72) Inventor: BRIGHT, Ben, Tewkesbury Gloucestershire GL20 6FL (GB); MAZUR, Ben, Ashley Down Bristol BS7 9QP (GB)
(74) Representative: Ponder, William Anthony John
(86) International application number: PCT/EP2016/077681
(87) International publication number: WO 2017/108268

(56) References cited:
- EP-A1- 1 618 803
- WO-A1-2013/050934
- WO-A1-2014/153515
- US-A1- 2008 257 915
- US-A1- 2015 020 822

## Description

The invention relates to an electrically operated aerosol-generating system, such as an electrically operating smoking system. In particular the invention relates to a system that actively pumps a liquid from a reservoir to a vapouriser.

Liquid based, electrically heated smoking systems are becoming increasingly popular. Typically these systems comprise a liquid store, an electric heater and a capillary wick that conveys liquid from the reservoir to the heater, together with a power supply and electric circuitry. The heater is typically a coil of wire wrapped around the capillary wick and operates by resistive heating. The liquid in the capillary wick is vapourised by the heater to form a vapour. A user puffs of the system causing air to flow past the heater. The airflow past the heater entrains the vapour and the vapour subsequently cools within the airflow to form an aerosol.

WO 2014/153515 A1 discloses an electronic smoking article which includes an outer housing, a micro pump system configured to pump a liquid material contained within a liquid supply reservoir through an outlet of the supply reservoir into a capillary. The capillary has an inlet and an outlet, the inlet being in communication with the outlet of the liquid supply reservoir.

WO 2015/112750 A1 discloses methods, devices, systems, and computer readable medium for delivering one or more compounds to a subject.

Typically, the liquid store may be provided as a refillable or replaceable cartridge that is fixed to, or inserted into, the remainder of the system. The cartridge may also include the wick and heater. Alternatively, the wick and heater may be provided in an atomiser assembly separate to the cartridge.

The liquid reservoir is typically a sealed enclosure and has a rigid housing. This is because it is very undesirable for liquid to leak from the reservoir before or during use. Aside from leakage being unpleasant and inconvenient for uses, the liquids used in electrically operated smoking systems may be irritating to the skin. Leakage of liquid into the device may also be damaging to the circuitry and may be hard to clean. So the liquid reservoir is typically a fully sealed and robust container.

However, the fixed volume of the liquid reservoir means that, as liquid is removed from the liquid reservoir, the pressure inside the reservoir drops. This pressure drop means that the liquid is less efficiently delivered to wick. In most systems, before the pressure drops very far below atmospheric pressure, air is able to enter the liquid reservoir through the wick in order to balance the pressure inside and outside of the reservoir. But this air flow through the wick decreases wicking performance and so may negatively affect aerosol density and flavour.

So, while the arrangement of liquid store and wick provides satisfactory delivery of liquid to the heater, it would be desirable to provide a more efficient and reliable mechanism for delivering liquid to a vapouriser in an electrically operated aerosol-generating system.

In a first aspect, there is provided an electrically operated aerosol-generating system comprising:
a liquid reservoir comprising a rigid housing; an air inlet valve in the rigid housing, the air inlet valve configured to allow air into the liquid reservoir when a pressure difference between outside of the housing and inside of the housing exceeds a threshold pressure difference; a vapouriser configured to vapourise the liquid; and a pump connected to an outlet through the rigid housing and configured to pump liquid from the liquid reservoir to the vapouriser.

The use of a pump between the liquid reservoir and the vapouriser improves the reliability and efficiency of delivery of liquid to the vapouriser. In addition, the invention provides an air inlet valve in the liquid reservoir. This allows the pressure inside the reservoir to equalise with atmospheric pressure. This in turn allows a rigid reservoir housing to be used, providing the necessary robustness for the liquid reservoir, particularly for a refillable reservoir without the problem of reduced pressure in the reservoir.

The liquid reservoir of the aerosol-generating system has a rigid housing. As used herein, the term 'rigid housing' is used to mean a housing that is self-supporting. The housing may be substantially cylindrical. An opening for the air inlet valve may be provided at one end of the cylinder. The housing of the liquid storage portion may have a substantially circular cross section.

The liquid reservoir may further comprise a carrier material within the housing for holding the liquid. The liquid may be adsorbed or otherwise loaded onto a carrier or support. The carrier material may be made from any suitable absorbent plug or body, for example, a foamed metal or plastics material, polypropylene, terylene, nylon fibres or ceramic.

The pump may be a micropump, such as a piezoelectric micropump.

The valve may be a check valve, such as a ball check valve or a duckbill check valve. The valve may be push fit to the housing of the liquid reservoir.

The system may comprise a capillary material positioned between the pump and the vapouriser. The capillary material may have a fibrous or spongy structure. The capillary material preferably comprises a bundle of capillaries. For example, the capillary material may comprise a plurality of fibres or threads or other fine bore tubes. The fibres or threads may be generally aligned to convey liquid to the heater. Alternatively, the capillary material may comprise sponge-like or foam-like material. The structure of the capillary material forms a plurality of small bores or tubes, through which the liquid can be transported by capillary action. The capillary material may comprise any suitable material or combination of materials. Examples of suitable materials are a sponge or foam material, ceramic- or graphite-based materials in the form of fibres or sintered powders, foamed metal or plastics materials, a fibrous material, for example made of spun or extruded fibres, such as cellulose acetate, polyester, or bonded polyolefin, polyethylene, terylene or polypropylene fibres, nylon fibres or ceramic. The capillary material may have any suitable capillarity and porosity so as to be used with different liquid physical properties. The liquid has physical properties, including but not limited to viscosity, surface tension, density, thermal conductivity, boiling point and vapour pressure, which allow the liquid to be transported through the capillary material by capillary action. The capillary material may be configured to convey the aerosol-forming substrate to the vapouriser. The capillary material may extend into interstices in the vapouriser.

The vapouriser may comprise an electrical heater. The electrical heater may be a coil of wire surrounding the capillary wick. Alternatively, the electrical heater may be a mesh heater. The mesh heater may be in contact with the capillary material. The electrical heater may be inductively heated or may be electrically connected to a power supply. Alternatively, the vapouriser may be a vibrating mesh or a vibrating diaphragm.

The system may comprise a power supply configured to supply power to operate the pump and the vapouriser. The power supply may be a battery. The battery may be a Lithium based battery, for example a Lithium-Cobalt, a Lithium-Iron-Phosphate, a Lithium Titanate or a Lithium-Polymer battery. The battery may be a Nickel-metal hydride battery or a Nickel cadmium battery. The power supply may be another form of charge storage device such as a capacitor. The power supply may require recharging and be configured for many cycles of charge and discharge. The power supply may have a capacity that allows for the storage of enough energy for one or more smoking experiences; for example, the power supply may have sufficient capacity to allow for the continuous generation of aerosol for a period of around six minutes, corresponding to the typical time taken to smoke a conventional cigarette, or for a period that is a multiple of six minutes. In another example, the power supply may have sufficient capacity to allow for a predetermined number of puffs or discrete activations of the heating means and actuating means.

The system may comprise electric circuitry. The electric circuitry may comprise a microprocessor, which may be a programmable microprocessor, a microcontroller, or an application specific integrated chip (ASIC) or other electronic circuitry capable of providing control. The electric circuitry may comprise further electronic components.

The electric circuitry may be configured to regulate a supply of power to the vapouriser .Power may be supplied to the vapouriser continuously following activation of the system or intermittently, such as on a puff-by-puff basis. The electric circuitry may be configured to regulate the supply of power to the pump.

The aerosol-generating system may comprise a puff detector in communication with the electric circuitry. The puff detector may be configured to detect when a user draws on the system. The electric circuitry may be configured to control power to the vapouriser in dependence on the input from the puff detector, or the pump, or both the pump and the vapouriser.

The aerosol-generating system may comprise a user input, such as a switch or button. This enables the user to turn the system on. The switch or button may activate the vapouriser.

The electric circuitry may be configured to operate the pump based on activation of the vapouriser. For example the pump may be activated when the vapouriser is activated. Alternatively the electric circuitry may be configured to operate the pump following activation of the vapouriser. For example, the vapouriser may be activated based on a sensed user puff or based on another user input. Activation of the vapouriser will deplete the liquid in the vicinity of the vapouriser. The pump may be controlled to supply more liquid to the vapouriser after a predetermined duration of activation of the vapouriser. The electric circuitry may also be configured to operate the pump when the system is switched on. If there has been a significant period of time since the last use of the system, the vapouriser may have become dry, and so it may be beneficial to operate the pump prior to activation of the vapouriser.

The system may be an electrically operated smoking system. The system may be a handheld aerosol-generating system. The aerosol-generating system may have a size comparable to a conventional cigar or cigarette. The smoking system may have a total length between approximately 30 mm and approximately 150 mm. The smoking system may have an external diameter between approximately 5 mm and approximately 30mm.

The liquid in the liquid reservoir may comprise a plant-based material. The liquid may comprise tobacco. The liquid may comprise nicotine. The liquid may comprise a tobacco-containing material containing volatile tobacco flavour compounds, which are released from the liquid upon heating. The liquid may alternatively comprise a non-tobacco-containing material. The liquid may comprise homogenised plant-based material. The liquid may comprise homogenised tobacco material. The liquid may comprise at least one aerosol-former. An aerosol-former is any suitable known compound or mixture of compounds that, in use, facilitates formation of a dense and stable aerosol and that is substantially resistant to thermal degradation at the temperature of operation of the system. Suitable aerosol-formers are well known in the art and include, but are not limited to: polyhydric alcohols, such as triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate. Preferred aerosol formers are polyhydric alcohols or mixtures thereof, such as triethylene glycol, 1,3-butanediol and, most preferred, glycerine. The liquid may comprise other additives and ingredients, such as flavourants.

In a second aspect, there is provided a cartridge for an electrically operated aerosol-generating system, comprising: a liquid reservoir comprising a rigid housing; an air inlet valve in the rigid housing, configured to allow air into the liquid reservoir when a pressure difference between outside of the housing and inside of the housing exceeds a threshold pressure difference; and an outlet through the rigid housing configured to engage a pump in the electrically operated aerosol-generating system.

The cartridge may comprise a filling port in the rigid housing through which liquid can pass into the liquid reservoir. The filling port may be sealed by a piercable septum or by a removable plug.

The outlet may be sealed prior to engagement with the pump. For example, the cartridge may comprise a peelable or piercable septum, foil or film that seals the outlet.

The cartridge may comprise the pump. The pump may be a piezoelectric micropump.

The cartridge may comprise a vapouriser. The pump may be connected between the liquid reservoir and the vapouriser and may be configured to pump liquid from the liquid reservoir to the vapouriser.

The vapouriser may comprise an electrical heater.The vapouriser may comprise a capillary material configured to convey the liquid from the pump to the electrical heater.

The valve may be a check valve, such as a ball check valve or a duckbill check valve.

The pump and vapouriser may be provided in an atomiser assembly separate to, but connectable with, the cartridge. The aerosol generating system may additionally comprise a main body comprising a power source and control circuitry. The main body may be connectable with the cartridge or an atomiser assembly.

Features described with reference to the first aspect of the invention may be applied to the second aspect of the invention.

The invention will be further described, by way of example only, with reference to the accompanying drawings, in which:
Figure 1 is a schematic illustration of a first embodiment of an aerosol-generating system according to the present invention;
Figure 2 is a schematic illustration of the components of a cartridge in accordance with the invention;
Figure 3 is a cross sectional illustration of the valve of Figure 2; and
Figure 4 is a schematic illustration of another embodiment of a cartridge in accordance with the invention.

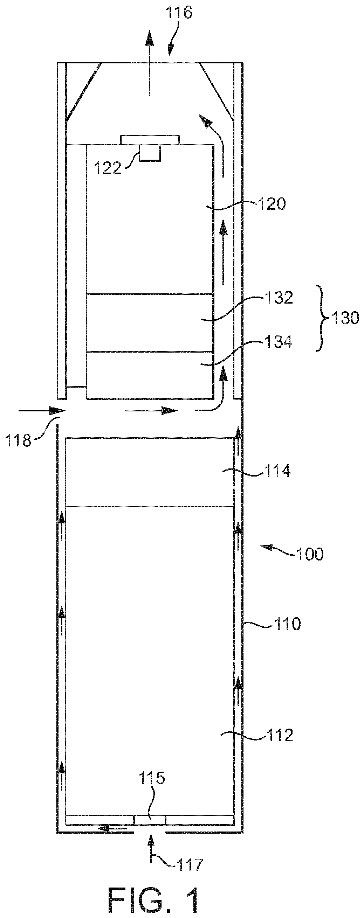

Figure 1 is an illustration of an aerosol-generating system according to the present invention. Figure 1 is schematic in nature. In particular, the components shown are not necessarily to scale either individually or relative to one another. The system is a handheld, electrically operated smoking device 100 and comprises a housing 110. Within the housing 110 there is an electric power supply in the form of battery 112 and control circuitry 114. Also within the housing there is a liquid reservoir 120 containing a liquid aerosol-forming substrate that is vapourised in order to form an aerosol that is inhaled by a user. An atomiser assembly 130 is provided within the housing, coupled to the liquid reservoir 120. The atomiser assembly comprises a vapouriser 134, in this example an electrical heater, and a pump 132 positioned to pump liquid from the liquid reservoir 120 to the vapouriser 134. Both the pump 132 and the electric heater 134 are provided with power from the battery 112 under the control of the control circuitry 114, as will be described.

The housing 110 includes an air inlet 118 and an air outlet 116. The air outlet 116 is provided at a mouthpiece end of the housing. In use, a user sucks on the mouthpiece end of the housing. This draws air through the air inlet 118 into the housing, past the vapouriser 134 and out through the outlet 116 into the user's mouth. The air drawn past the vapouriser entrains vapourised aerosol-forming substrate. The vapourised aerosol-forming substrate cools to form an aerosol as it moves through the device and into the user's mouth.

Activation of the heater may be controlled directly by a user pressing a button on the housing 110. Alternatively, the system may comprise an airflow sensor, such as a microphone 115, that detects airflow through the system and the heater may be activated based on signals from the airflow sensor. When a user draws air through the system, herein referred to as puffing, air flows past the air flow sensor 115. If the airflow detected by the airflow sensor exceeds a threshold value, then the control circuitry may activate the heater by supplying power to the heater. The control circuitry may supply power to the heater for a predetermined time period or may supply power to the heater for as long as the detected airflow exceeds a threshold. The control circuitry may include temperature sensing means, such as a dedicated temperature sensor or by monitoring an electrical resistance of the heater. The control circuitry may then supply power to the heater to raise the temperature of the heater to within a desired temperature range. The temperature should be sufficient to vapourise the aerosol-forming substrate but not so high that there is a significant risk of combustion.

The pump may be activated in the same way as the heater. For example the control circuitry may supply power to the pump for the same time periods as power is supplied to the heater. Alternatively, the control circuitry may supply power to the pump in periods immediately following activation of the heater.

The liquid in this example comprises a mixture of water, glycerol, propylene glycol, nicotine and flavourings. The liquid is held within the liquid reservoir 120. The liquid reservoir is provided as a cartridge that can be replaced when the liquid has been used up. In order to prevent leakage of the liquid, both before and during use, the liquid reservoir has a housing formed from a rigid plastics material, and is liquid tight. As used herein "rigid" means that the housing that is self-supporting. In this example, the reservoir is formed by 3D printing using an acrylic based photopolymer. The cartridge needs to be robust and able to withstand significant loads during shipping and storage. However, because the liquid reservoir housing is sealed and rigid, the liquid reservoir has a fixed internal volume. A reduction in the internal pressure inside the liquid reservoir as liquid is removed by the pump, could detrimentally affect the ability to pump liquid out of the reservoir. In order to prevent a significant drop in pressure, the liquid reservoir has an equalising air inlet valve 122. The equalising valve 122 allows air into the liquid reservoir when the pressure difference between the inside the reservoir and outside of the reservoir exceeds a threshold pressure difference.

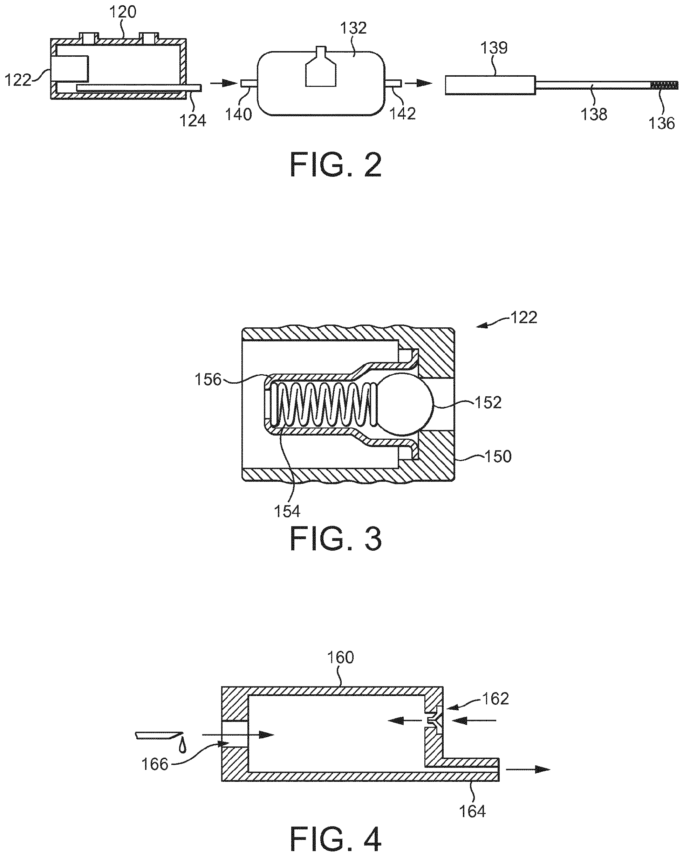

Figure 2 is an exploded view of the liquid reservoir 120, pump 132 and a heater assembly, the heater assembly comprising tube 139, a capillary nozzle 138 and a heater 136. As shown in Figure 2 the liquid reservoir comprises an equalising valve 122 and a liquid outlet 124. The liquid outlet 124 is configured to engage with an inlet 140 of the pump 132. The outlet 124 may be sealed with a removable cap prior to use or may be sealed with a piercable seal.

The pump is a piezoelectric micropump, such as an MP6 pump from Bartels Mikrotechnik GmbH, Konrad-Adenauer-Allee 11, 44263 Dortmund, Germany (www.bartelsmikrotechnik.de). The pump has an outlet 142 that engages the tube 139 of the heater assembly.

The tube 139 connects the pump 132 to a capillary nozzle 138. The capillary nozzle 138 is a 2ml glass capillary. A nickel-chromium heater wire is wound around the capillary nozzle to heat liquid in the nozzle.

Figure 3 shows the construction of the check valve 122 of Figure 2 in detail. The valve 122 is a 5mm diameter, ceramic ball check valve, from The Lee Company, 2 Pettipaug Rd, PO Box 424, Westbrook, CT 06498-0424 USA. The valve 122 is designed to be push fit to the reservoir housing. The valve comprises a stainless steel body 150 and a stainless steel frame 156. A stainless steel spring 154, seated on the frame 156, urges the ceramic ball 152 against the body to seal an aperture in the body. When the pressure difference across the ball becomes great enough to move the ball against the bias of the spring, the aperture is unsealed, allowing air into the liquid reservoir 120.

The liquid reservoir 120 is provided as a cartridge in this example. The cartridge is received in the housing 110 of the system. The cartridge may be keyed to ensure that the outlet 124 correctly engages the inlet 140 of the pump. The pump 132 and heater assembly 134 are provided as a replaceable atomiser assembly 130. The atomiser assembly is received in the housing 110 but alternatively may form part of the housing 110.

The battery 112 is a lithium iron phosphate battery that is rechargeable. Charging contacts are provided on the housing 112. The electric circuitry 114 includes a programmable microprocessor that is configured to control the supply of power to the heater 134 and to the pump 132.

The housing 110 is formed from polyetheretherketone (PEEK) and has a size and shape that is comfortable for a user to hold in a single hand. A removable mouthpiece may be provided around the air outlet 116. The mouthpiece may be removed to allow access to a cavity in the housing in which the cartridge is held.

A user assembles the main housing, the atomiser assembly and the liquid reservoir cartridge together prior to operation. In order to activate the system, the user presses a button on the housing 110. In this embodiment, following a user activating the device, the electric circuitry supplies power to the pump 132 so that liquid is pumped to the heater assembly 134. The user then puffs on the mouthpiece of the device, drawing air through the device. The device includes a puff sensor 115, which is a microphone, that detects the flow of air through the device. The air flow for the puff sensor enters the system through an auxiliary air inlet 117 much smaller than air inlet 118. In response to a signal from the microphone 115, the electric circuitry supplies power to the heater 136 so that the heater heats up and vapourises the liquid in the capillary nozzle 138. The vapourised liquid substrate then cools in the airflow and condenses to form an aerosol that is drawn out of the system and into the user's mouth. In this example, the electric circuitry supplies power to the heater for a fixed duration following detection of a puff. However, other control schemes for the supply of power to the heater may be used. Power is supplied to the pump for the same period that power is supplied to the heater in order to replenish the liquid in the nozzle 138 as it is being vapourised. When a user has finished using the system they can switch the system off using a button. Additionally, the electric circuitry may be configured to switch the system off if no user puffs are detected for determined time period.

As liquid is drawn out of the liquid reservoir by the pump, the valve 122 opens to equalise the pressure inside the reservoir with the pressure outside of the reservoir. The valve may be provided with a covering baffle to reduce any flow of aerosol back into the reservoir during a user puff.

The embodiment described with reference to Figures 2 and 3 is just one example of a system in accordance with the invention. Figure 4 shows an alternative liquid reservoir and valve arrangement. The liquid reservoir of Figure 4 is a refillable reservoir. The liquid reservoir shown in figure 4 has a rigid, generally cylindrical housing 160 and has a liquid outlet 164 configured to engage a pump and an equalising valve 162. The equalising valve in the embodiment of Figure 4 is a duckbill valve designed to allow air into the liquid reservoir when the pressure difference between inside the reservoir and outside the reservoir exceeds a threshold pressure difference. The liquid reservoir also includes a filling port through which the liquid reservoir can be refilled with liquid aerosol-forming substrate. The filling port is sealed by an elastomeric septum 166. In order to refill the liquid reservoir, the septum 166 is pierced by a needle and liquid injected through the needle into the liquid reservoir.

It is also possible to use alternative vaporisers to the heater described with reference to Figure 2. For example, the vapouriser may be a heated mesh or a vibrating mesh, with the pump arranged to deliver liquid to the interstices of the mesh. The vapouriser may be a heated plate or pair of plates and the pump may deliver liquid to the plate or plates. The vaporiser may be an inductively heated element.

It is also possible to use an alternative arrangement of the components shown in Figure 1 and different airflow paths through the system. For example, the atomiser may be closer to the mouthpiece of the system than the liquid reservoir. The system may also comprise air inlets and the puff sensor in different positions.

## Claims

1. A cartridge for an electrically operated aerosol-generating system, comprising:
a liquid reservoir (120) comprising a rigid housing (110);
an air inlet valve (122) in the rigid housing, configured to allow air into the liquid reservoir when a pressure difference between outside of the housing and inside of the housing exceeds a threshold pressure difference; and
an outlet (164) through the rigid housing configured to engage a pump (132) in the electrically operated aerosol-generating system.

2. A cartridge according to claim 1, comprising a filling port in the rigid housing (110) through which liquid can pass into the liquid reservoir (120).

3. A cartridge according to claim 1 or 2, wherein the outlet (164) is sealed prior to engagement with the pump (132).

4. A cartridge according to any one of the preceding claims, wherein the cartridge comprises the pump (132).

5. A cartridge according to claim 4, wherein the cartridge comprises a vapouriser (134), and wherein the pump (132) is connected between the liquid reservoir (120) and the vapouriser and configured to pump liquid from the liquid reservoir to the vapouriser.

6. A cartridge according to claim 5, wherein the vapouriser (134) comprises an electrical heater.

7. A cartridge according to claim 6, wherein the vapouriser (134) comprises a capillary material configured to convey the liquid to the electrical heater.

8. A cartridge according to any one of the preceding claims, wherein the pump (132) is a piezoelectric micropump.

9. A cartridge according to any one of the preceding claims, wherein the valve (122) is a check valve, such as a ball check valve or a duckbill check valve.

10. An electrically operated aerosol-generating system comprising:
a liquid reservoir (120) comprising a rigid housing (110);
an air inlet valve (122) in the rigid housing, configured to allow air into the liquid reservoir when a pressure difference between outside of the housing and inside of the housing exceeds a threshold pressure difference;
a vapouriser (134) configured to vapourise the liquid; and
a pump (132) connected to an outlet (164) through the rigid housing and configured to pump liquid from the liquid reservoir to the vapouriser.

11. An electrically operated aerosol-generating system according to claim 10, wherein the pump (132) is a piezoelectric micropump.

12. An electrically operated aerosol-generating system according to claim 10 or 11 wherein the vapouriser (134) comprises an electrical heater.

13. An electrically operated aerosol-generating system according to claim 10, 11 or 12, wherein the system comprises a power supply configured to supply power to operate the pump (132) and the vapouriser (134).

14. An electrically operated aerosol-generating system according to any one of claims 10 to 13, comprising a controller configured to operate the pump (132) when the vapouriser (134) is activated.

15. An electrically operated aerosol-generating system according to any one of claims 10 to 14, wherein the system is a hand held electrically operated smoking device.

## Patentansprüche

1. Patrone für ein elektrisch betriebenes Aerosolerzeugungssystem, aufweisend:
einen Flüssigkeitsvorratsbehälter (120), aufweisend ein starres Gehäuse (110);
ein Lufteinlassventil (122) in dem starren Gehäuse, ausgelegt zum Einlassen von Luft in den Flüssigkeitsvorratsbehälter,
wenn eine Druckdifferenz zwischen der Außenseite des Gehäuses und der Innenseite des Gehäuses eine Schwellendruckdifferenz überschreitet; und
einen Auslass (164) durch das starre Gehäuse, der zum Eingriff mit einer Pumpe (132) in dem elektrisch betriebenen Aerosolerzeugungssystem ausgelegt ist.

2. Patrone nach Anspruch 1, aufweisend eine Einfüllöffnung in dem starren Gehäuse (110), durch die Flüssigkeit in den Flüssigkeitsvorratsbehälter (120) strömen kann.

3. Patrone nach Anspruch 1 oder 2, wobei der Auslass (164) vor dem Eingriff mit der Pumpe (132) abgedichtet ist.

4. Patrone nach einem der vorhergehenden Ansprüche, wobei die Patrone die Pumpe (132) aufweist.

5. Patrone nach Anspruch 4, wobei die Patrone einen Verdampfer (134) aufweist und wobei die Pumpe (132) zwischen dem Flüssigkeitsvorratsbehälter (120) und dem Verdampfer verbunden und zum Pumpen von Flüssigkeit aus dem Flüssigkeitsvorratsbehälter zu dem Verdampfer ausgelegt ist.

6. Patrone nach Anspruch 5, wobei der Verdampfer (134) eine elektrische Heizvorrichtung aufweist.

7. Patrone nach Anspruch 6, wobei der Verdampfer (134) ein Kapillarmaterial aufweist, das zum Fördern der Flüssigkeit zu der elektrischen Heizvorrichtung ausgelegt ist.

8. Patrone nach einem der vorhergehenden Ansprüche, wobei die Pumpe (132) eine piezoelektrische Mikropumpe ist.

9. Patrone nach einem der vorhergehenden Ansprüche, wobei das Ventil (122) ein Rückschlagventil, wie beispielsweise ein Kugelrückschlagventil oder ein Entenschnabelrückschlagventil, ist.

10. Elektrisch betriebenes Aerosolerzeugungssystem, aufweisend:
einen Flüssigkeitsvorratsbehälter (120), aufweisend ein starres Gehäuse (110);
ein Lufteinlassventil (122) in dem starren Gehäuse, ausgelegt zum Einlassen von Luft in den Flüssigkeitsvorratsbehälter,
wenn eine Druckdifferenz zwischen der Außenseite des Gehäuses und der Innenseite des Gehäuses eine Schwellendruckdifferenz überschreitet;
einen Verdampfer (134), ausgelegt zum Verdampfen der Flüssigkeit; und
eine Pumpe (132), die mit einem Auslass (164) durch das starre Gehäuse verbunden und zum Pumpen von Flüssigkeit aus dem Flüssigkeitsvorratsbehälter zu dem Verdampfer ausgelegt ist.

11. Elektrisch betriebenes Aerosolerzeugungssystem nach Anspruch 10, wobei die Pumpe (132) eine piezoelektrische Mikropumpe ist.

12. Elektrisch betriebenes Aerosolerzeugungssystem nach Anspruch 10 oder 11, wobei der Verdampfer (134) eine elektrische Heizvorrichtung aufweist.

13. Elektrisch betriebenes Aerosolerzeugungssystem nach Anspruch 10, 11 oder 12, wobei das System eine Energieversorgung aufweist, die zum Bereitstellen von Energie zum Betreiben der Pumpe (132) und des Verdampfers (134) ausgelegt ist.

14. Elektrisch betriebenes Aerosolerzeugungssystem nach einem der Ansprüche 10 bis 13, aufweisend eine Steuerung, die zum Betreiben der Pumpe (132) bei aktiviertem Verdampfer (134) ausgelegt ist.

15. Elektrisch betriebenes Aerosolerzeugungssystem nach einem der Ansprüche 10 bis 14, wobei das System eine handgehaltene elektrisch betriebene Vorrichtung zum Rauchen ist.

## Revendications

1. Cartouche pour un système de génération d'aérosol à fonctionnement électrique, comprenant :
un réservoir de liquide (120) comprenant un logement rigide (110) ;
un clapet d'entrée d'air (122) dans le logement rigide, configuré pour permettre l'entrée d'air dans le réservoir de liquide lorsqu'une différence de pression entre l'extérieur du logement et l'intérieur du logement dépasse une différence de pression seuil ; et
une sortie (164) à travers le logement rigide configurée pour mettre en prise une pompe (132) dans le système de génération d'aérosol à fonctionnement électrique.

2. Cartouche selon la revendication 1, comprenant un orifice de remplissage dans le logement rigide (110) à travers lequel le liquide peut passer dans le réservoir de liquide (120).

3. Cartouche selon la revendication 1 ou 2, dans laquelle la sortie (164) est scellée avant la mise en prise avec la pompe (132) .

4. Cartouche selon l'une quelconque des revendications précédentes, dans laquelle la cartouche comprend la pompe (132) .

5. Cartouche selon la revendication 4, dans laquelle la cartouche comprend un vaporisateur (134), et dans laquelle la pompe (132) est raccordée entre le réservoir de liquide (120) et le vaporisateur et configurée pour pomper du liquide du réservoir de liquide vers le vaporisateur.

6. Cartouche selon la revendication 5, dans laquelle le vaporisateur (134) comprend un dispositif de chauffage électrique.

7. Cartouche selon la revendication 6, dans laquelle le vaporisateur (134) comprend une matière capillaire configurée pour transporter le liquide vers le dispositif de chauffage électrique.

8. Cartouche selon l'une quelconque des revendications précédentes, dans laquelle la pompe (132) est une micropompe piézoélectrique.

9. Cartouche selon l'une quelconque des revendications précédentes, dans laquelle le clapet (122) est un clapet de retenue, tel qu'un clapet de retenue à bille ou un clapet de retenue à bec de canard.

10. Système de génération d'aérosol à fonctionnement électrique comprenant :
un réservoir de liquide (120) comprenant un logement rigide (110) ;
un clapet d'entrée d'air (122) dans le logement rigide, configuré pour permettre l'entrée d'air dans le réservoir de liquide lorsqu'une différence de pression entre l'extérieur du logement et l'intérieur du logement dépasse une différence de pression seuil ;
un vaporisateur (134) configuré pour vaporiser le liquide ; et
une pompe (132) raccordée à une sortie (164) à travers le logement rigide et configurée pour pomper du liquide du réservoir de liquide vers le vaporisateur.

11. Système de génération d'aérosol à fonctionnement électrique selon la revendication 10, dans lequel la pompe (132) est une micropompe piézoélectrique.

12. Système de génération d'aérosol à fonctionnement électrique selon la revendication 10 ou 11, dans lequel le vaporisateur (134) comprend un dispositif de chauffage électrique.

13. Système de génération d'aérosol à fonctionnement électrique selon la revendication 10, 11 ou 12, dans lequel le système comprend une alimentation électrique configurée pour fournir de l'énergie afin de faire fonctionner la pompe (132) et le vaporisateur (134).

14. Système de génération d'aérosol à fonctionnement électrique selon l'une quelconque des revendications 10 à 13, comprenant un contrôleur configuré pour faire fonctionner la pompe (132) lorsque le vaporisateur (134) est activé.

15. Système de génération d'aérosol à fonctionnement électrique selon l'une quelconque des revendications 10 à 14, dans lequel le système est un dispositif à fumer à fonctionnement électrique portatif.
